(19) Europäisches Patentamt
European Patent Office
Office européen des brevets

(11) **EP 3 479 754 A1**

(12) **EUROPEAN PATENT APPLICATION**

(43) Date of publication:
08.05.2019 Bulletin 2019/19

(51) Int Cl.:
**A61B 5/00** (2006.01)    **A61B 5/024** (2006.01)
**A61B 5/1455** (2006.01)

(21) Application number: 17199495.7

(22) Date of filing: 01.11.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventor: **DE HAAN, Gerard**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DEVICE, SYSTEM AND METHOD FOR DETERMINING AT LEAST ONE VITAL SIGN OF A SUBJECT**

(57)    The present invention relates to a device, system and method for determining at least one vital sign of a subject. The device (130) comprises an input interface (131) configured to obtain at least three detection signals (210) derived from detected electromagnetic radiation transmitted through or reflected from a skin region of a subject, wherein each detection signal comprises wavelength-dependent reflection or transmission information in a different wavelength channel, a vital sign extraction unit (132) configured to extract multiple candidate vital signs (232) of the same type of vital sign from said at least three detection signals, wherein each candidate vital sign is extracted from a different detection signal or a different combination of at least two detection signals, a vital sign determination unit (133) configured to determine a final vital sign (233) from said multiple candidate vital signs, and a reliability information unit (134) configured to provide reliability information (234a) indicating the reliability of said final vital sign and/or one or more of said candidate vital signs and to provide unreliability source information (234b) indicating the source of unreliability of said final vital sign and/or one or more of said candidate vital signs.

FIG.2

**Description**

FIELD OF THE INVENTION

The present invention relates to a device, system and method for determining at least one vital sign of a subject.

BACKGROUND OF THE INVENTION

Vital signs of a person, for example the heart rate (HR), the respiration rate (RR) or the arterial blood oxygen saturation (SpO2), serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform (also called PPG signal) can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation of a subject are attached to the skin of the subject, for instance to a fingertip, earlobe or forehead. Therefore, they are referred to as 'contact' PPG devices. Although contact PPG is regarded as a basically non-invasive technique, contact PPG measurement is often experienced as being unpleasant and obtrusive, since the pulse oximeter is directly attached to the subject and any cables limit the freedom to move and might hinder a workflow.

Non-contact, remote PPG (rPPG) devices (also called camera-based device) for unobtrusive measurements have been proposed in the last decade. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications.

Using PPG technology, vital signs can be measured, which are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or high required accuracy of the application, where an improved robustness and accuracy of the vital sign measurement devices and methods is required, particularly for the more critical healthcare applications.

Further, unexpected blood substances, in particular blood gasses like CO or MetHB, may lead to poor SNR or miscalibration of the method used for measurement or evaluation of the measurement leading to unreliable results.

Hence, there is a need for an improved device, system and method for determining at least one vital sign of a subject to obtain results with higher reliability even in case of motion and presence of unexpected blood substances.

SUMMARY OF THE INVENTION

It is an object of the present invention to provide a device, system and method for determining at least one vital sign of a subject by which results with higher reliability even in case of motion and presence of unexpected blood substances can be achieved.

In a first aspect of the present invention a device for determining at least one vital sign of a subject is presented comprising:

- an input interface configured to obtain at least three detection signals derived from detected electromagnetic radiation transmitted through or reflected from a skin region of a subject, wherein each detection signal comprises wavelength-dependent reflection or transmission information in a different wavelength channel,
- a vital sign extraction unit configured to extract multiple candidate vital signs of the same type of vital sign from said at least three detection signals, wherein each candidate vital sign is extracted from a different detection signal or a different combination of at least two detection signals,

- a vital sign determination unit configured to determine a final vital sign from said multiple candidate vital signs, and
- a reliability information unit configured to provide reliability information indicating the reliability of said final vital sign and/or one or more of said candidate vital signs and to provide unreliability source information indicating the source of unreliability of said final vital sign and/or one or more of said candidate vital signs.

In a further aspect of the present invention a system for determining at least one vital sign of a subject is presented comprising:

- a detector for detecting electromagnetic radiation transmitted through or reflected from a skin region of a subject and for deriving at least three detection signals from the detected electromagnetic radiation, wherein each detection signal comprises wavelength-dependent reflection or transmission information in a different wavelength channel, and
- a device as disclosed herein for determining at least one vital sign of the subject from the at least three detection signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to exploit a redundancy in multi-wavelength PPG detectors. Particularly, measurements with multiple wavelengths exhibit differences in behavior during patient movements and during presence of unexpected blood substances. This allows detecting situations, in which the reliability of measurements and/or of a determined vital sign is reduced, and getting information on the quantity of the reliability and on the source of any potential unreliability. This makes it possible that alarms are only issued in situations with reliable results, and/or that false alarms are prevented in situations with unreliable results, and that miscalibrations and/or misinterpretations are made in case other sources like the presence of subject motion and/or the presence of blood gasses influence the measurements.

According to an embodiment said reliability information unit is configured to provide unreliability source information indicating one or more of subject motion, unexpected blood gasses, blood species and blood composition. With this kind of information, for instance, the measurement and/or the evaluation used e.g. for determining SpO2 as a ratio of pulsatilities (AC signal values) of two different wavelength channels, can be improved.

In another embodiment the device further comprises a pulse signal computation unit configured to compute multiple pulse signals from said at least three detection signals, wherein each pulse signal is extracted from a different detection signal or a different combination of at least two detection signals. These pulse signals are preferably computed, as proposed in a further embodiment, from said at least three detection signals using a blood-volume vector based PBV or adaptive PBV method. Further, the pulse signal computation unit may be configured to compute said multiple pulse signals from said at least three detection signals using different signature vectors for the computation of each pulse signal, said signature vectors providing an expected relative strength of the pulse signal in the at least three detection signals, wherein the computation of a pulse signal involves a weighted combination at least two detection signals using weights selected such that the resulting pulse signal correlates with the original detection signals as indicated by the respective signature vector.

As explained above, a PPG signal results from variations of the blood volume in the skin. Hence, the variations give a characteristic pulsatility "signature" when viewed in different spectral components of the reflected/transmitted light. This "signature is basically resulting as the contrast (difference) of the absorption spectra of the blood and that of the blood-less skin tissue. If the detector, e.g. a camera or sensor, has a discrete number of color channels, each sensing a particular part of the light spectrum, then the relative pulsatilities in these channels can be arranged in a "signature vector", also referred to as the "normalized blood-volume vector", PBV. It has been shown in G. de Haan and A. van Leest, "Improved motion robustness of remote-PPG by using the blood volume pulse signature", Physiol. Meas. 35 1913, 2014, which is herein incorporated by reference, that if this signature vector is known then a motion-robust pulse signal extraction on the basis of the color channels and the signature vector is possible. For the quality of the pulse signal it is essential though that the signature is correct, as otherwise the known methods mixes noise into the output pulse signal in order to achieve the prescribed correlation of the pulse vector with the normalized color channels as indicated by the signature vector.

Details of the PBV method and the use of the normalized blood volume vector (called "predetermined index element having a set orientation indicative of a reference physiological information") have also been described in US 2013/271591 A1, which details are also herein incorporated by reference.

The characteristic wavelength-dependency of the PPG signal varies when the composition of the blood changes. Particularly, oxygen-saturation of arterial blood has a strong effect on the absorption in the wavelength range between 620nm and 780nm. This changing signature for different SpO2 values leads to relative PPG pulsatility that depends on the arterial blood oxygenation. This dependency can be used to realize a motion-robust remote SpO2 monitoring system that has been named adaptive PBV method (aPBV) and is described in detail in M. van Gastel, S. Stuijk and G. de Haan, "New principle for measuring arterial blood oxygenation, enabling motion-robust remote monitoring", Nature Scientific Reports, Nov. 2016. The description of the details of the aPBV method in this document is also herein incorporated by reference.

In another embodiment said reliability information unit is configured to determine said reliability information and said unreliability source information by comparing said pulse signals. For instance, based on the similarity of said pulse signals according to a pulse signal similarity metric, in particular in the frequency domain, or according to the SNR, or according to the low mean squared difference between pairs of pulse signals, said reliability information and said unreliability source information can be obtained.

An embodiment is directed to the determination of arterial blood oxygen saturation, which is an important vital sign. In this embodiment said vital sign extraction unit is configured to extract multiple candidate SpO2 values as candidate vital signs from different combinations of at least two detection signals, wherein said vital sign determination unit is configured to determine the final SpO2 value from said multiple candidate SpO2 values, and wherein said reliability information unit is configured to determine said reliability information and said unreliability source information based on the similarity of said candidate SpO2 values according to an SpO2 similarity metric, in particular according to the standard deviation of candidate SpO2 values, optionally filtered over a time interval, or according to SNR values of pulse signals extracted from said at least two detection signals by different versions of the adaptive PBV method, or according to the similarity of the candidate SpO2 values in combination with the strength of a motion signal representative for subject motion.

The proposed device may further comprise an output unit configured to output the final vital sign along with the reliability information and/or the unreliability source information. The output unit may e.g. be a user interface like a display, computer or loudspeaker.

Still further, the proposed device may comprise a control unit configured to generate, based on the final vital sign and based on the reliability information and/or the unreliability source information, an alarm control signal for controlling an alarm unit configured to issue an alarm and to output the generated alarm control signal. This provides the ability that, as proposed in a further embodiment, the control unit is configured to generate an alarm control signal that suppresses an alarm in case of low reliability of the final vital sign and/or to generate an alarm control signal that triggers an alarm indicating that unexpected blood gasses and/or subject motion caused a low reliability of the final vital sign.

Hereby, the control unit may be configured to use a metric based on dissimilarity of the candidate vital signs in combination with low probability of subject motion, which further supports the aim to prevent false alarm and to issue correct alarms with higher reliability.

BRIEF DESCRIPTION OF THE DRAWINGS

These and other aspects of the invention will be apparent from and elucidated with reference to the embodiment(s) described hereinafter. In the following drawings

Fig. 1 shows a diagram of the relative PPG amplitude in three different wavelengths;
Fig. 2 shows a diagram of the absorption spectrum of blood with different blood components;
Fig. 3 shows a diagram illustrating the effect of carbon-monoxide (CO) on the calibration curve for SpO2;
Fig. 4 shows a schematic diagram of an embodiment of a system according to the present invention;
Fig. 5 shows a schematic diagram of a first embodiment of a device according to the present invention;
Fig. 6 shows a diagram illustrating SpO2 measurements using the aPBV method;
Fig. 7 shows a diagram illustrating the standard deviation of three measurements of SpO2; and
Fig. 8 shows a schematic diagram of a second embodiment of a device according to the present invention.

DETAILED DESCRIPTION OF EMBODIMENTS

Recently, significant improvements to the motion robustness of remote PPG monitoring technique have been achieved. The essential insight enabling separation of motion-induced signal variations and the actual PPG variations is that motion affects all wavelength channels (typical PPG monitoring systems use at least two wavelength channels) equally, i.e. the relative signal strength of motion-induced variation is identical in all channels while the PPG-based variations are clearly wavelength dependent. The wavelength-dependency of the PPG signal is mainly caused by the absorption-contrast between blood and skin that varies with the wavelength. In addition it also depends to a minor extent on the penetration depth, and scattering properties of light in human skin that vary with the wavelength.

The characteristic wavelength-dependency of the PPG signal can be used as a signature, enabling motion-robust PPG measurement. The number of independent distortions that can be suppressed equals the number of wavelength channels minus one. This so-called PBV method is described in the above cited paper of G. de Haan and A. van Leest, "Improved motion robustness of remote-PPG by using the blood volume pulse signature", Physiol. Meas. 35 1913, 2014.

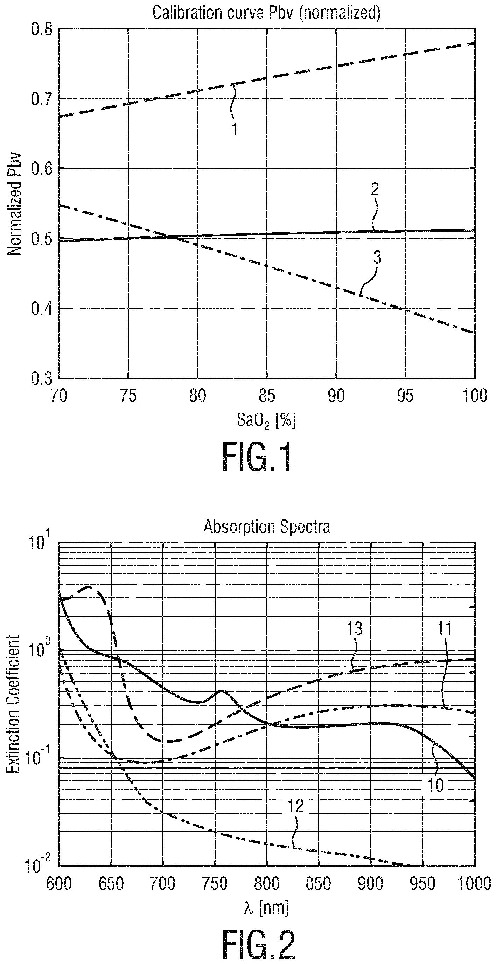

The characteristic wavelength-dependency of the PPG signal varies when the composition of the blood changes. Particularly, oxygen saturation of arterial blood has a strong effect on the absorption in the wavelength range between 620nm and 780nm. This changing signature for different SpO2 values leads to relative PPG pulsatility that depends on the arterial blood oxygenation (SaO2) as illustrated in Fig. 1 for three wavelengths in near infrared, in particular for $\lambda_1$=760nm (signal line 1), $\lambda_2$=800nm (signal line 2) and $\lambda_3$=905nm (signal line 3). This dependency can be used to realize a motion-robust remote SpO2 monitoring system that has been named adaptive PBV method (aPBV) and is described in the above cited paper of M. van Gastel, S. Stuijk and G. de Haan, "New principle for measuring arterial blood oxygenation, enabling motion-robust remote monitoring", Nature Scientific Reports, Nov. 2016.

It shall be noted here that SaO2 is the actual arterial oxygenation level as would be measured invasively by drawing blood and subsequent analysis. SpO2 is the result from an optical measurement, which is no more than a substitute for the actual measurement, which, however, has been proven to be clinically relevant under certain conditions, be it less accurate, i.e. SpO2 is the estimation result using photoplethysmography.

A problem with both the PBV method and the aPBV method is that they rely on assumed knowledge of this signature (possibly for different SpO2 values). This prior knowledge is threatened by unexpected blood-substances, like CO or MetHB, which may lead to poor SNR in the PBV method or miscalibration for the aPBV method. In these cases the measurement is unreliable.

Fig. 2 illustrates how other possible blood components may affect the blood absorption spectrum due to their deviant absorption spectrum. It is shown there that not only oxygenation of Hb (signal line 10) to HbO2 (signal line 11) leads to a different absorption spectrum of the blood. Also other possible blood components like carbonmonoxide (CO; signal line 12 for COHb) and methemoglobin (MetHB; signal line 13) change the absorption and consequently also the relative PPG amplitude (signature) at different wavelengths.

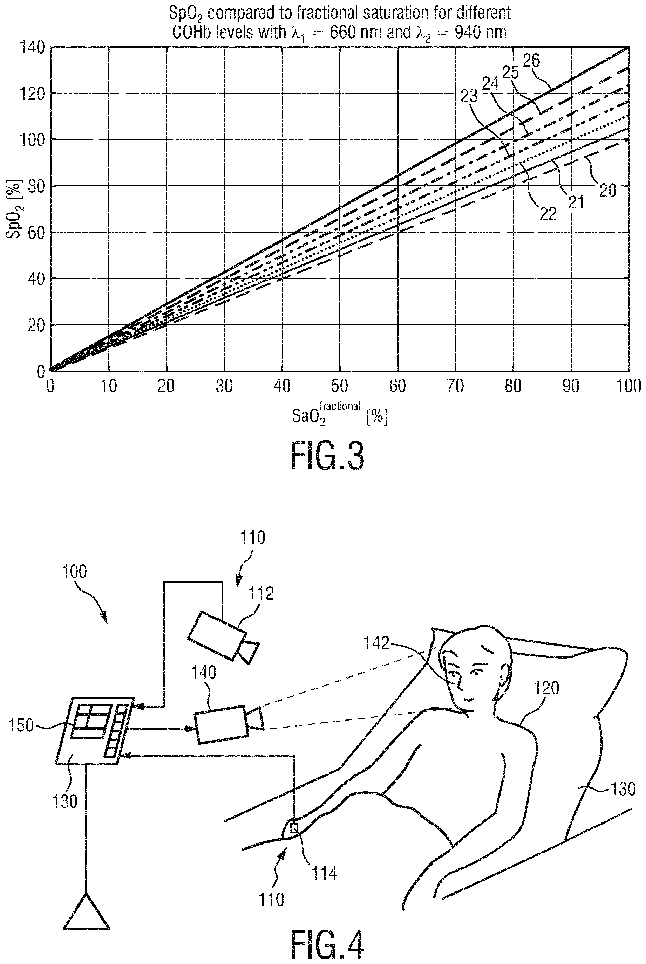

Consequently, (camera-based) vital signs monitoring methods using photoplethysmography generally produce a value for the monitored vital signs, even in cases where they cannot be reliably established. Examples where this may happen include cases where patient movements cause misdetection, but also less trivial situations in which other blood components (like CO and MetHB) cause offsets in the calibration curve of SpO2 measurements and modified blood-volume pulse signatures that cause impaired signal-to-noise ratio in output PPG-based vital signs (e.g. pulse and respiration signals). An example of the effect that CO has on the calibration curve of an SpO2 measurement using two NIR wavelengths is shown in Fig. 3. Here, signal line 20 is the regular curve (i.e. for COHb=0%), assuming absence of CO. The other signal lines 21-26 show the deviations for different concentrations of CO in the blood (in increasing steps of 5% from COHb=5% for signal line 21 up to COHb=30% for signal line 26).

A somewhat comparable unreliability problem occurs if the, e.g. motion-induced, distortions are so strong that they exceed the robustness of the PPG measurement method. In these cases typically the measured SpO2 equals the value that normally occurs for an oxygenation level where the pulsatilities in the wavelengths are identical. This value depends on the wavelengths used.

The present invention allows detection of such situations and quantification of the reliability of the vital sign enabling alarms for such cases or prevention of false alarms in case of temporary cases (movements). Vital signs in this context may include one or more of heart rate (HR), respiration rate (RR), the arterial blood oxygen saturation (SpO2), CO, CO2, MetHB, blood-pressure, glucose-levels, results from pulse-wave-form analysis (e.g. augmentation index), pulse-transit time, etc.

Fig. 4 shows a schematic diagram of an embodiment of a system 100 according to the present invention. The system 100 comprises a detector 110 for detecting electromagnetic radiation transmitted through or reflected from a skin region of a subject 120 and for deriving at least three detection signals 210 derived from the detected electromagnetic. Each detection signal 210 comprises wavelength-dependent reflection or transmission information in a different wavelength channel. The system 100 further comprises a device 130 for determining at least one vital sign of the subject from the at least three detection signals 210. The subject 120, in this example a patient, lies in a bed 130, e.g. in a hospital or other healthcare facility, but may also be a neonate or premature infant, e.g. lying in an incubator, or person at home or in a different environment.

There exist different embodiments for a detector for detecting electromagnetic radiation transmitted through or reflected from a subject, which may alternatively (which is preferred) or together be used. In the embodiment of the system 100 two different embodiments of the detector are shown and will be explained below. Both embodiments of the detector are configured for deriving detection signals from the detected electromagnetic radiation, wherein each detection signal comprises wavelength-dependent reflection or transmission information in a different wavelength channel. Herby, optical filters may be used which are preferably different, but their filter bandwidth can be overlapping. It is sufficient if their wavelength-dependent transmission is different.

In one embodiment the detector comprises a camera 112 (also referred to as imaging unit, or as camera-based or remote PPG sensor) including a suitable photosensor for (remotely and unobtrusively) capturing image frames of the subject 120, in particular for acquiring a sequence of image frames of the subject 120 over time, from which photoplethysmography signals can be derived. The image frames captured by the camera 112 may particularly correspond to a video sequence captured by means of an analog or digital photosensor, e.g. in a (digital) camera. Such a camera 112 usually includes a photosensor, such as a CMOS or CCD sensor, which may also operate in a specific spectral range (visible, IR) or provide information for different spectral ranges. The camera 112 may provide an analog or digital signal. The image frames include a plurality of image pixels having associated pixel values. Particularly, the image frames include pixels representing light intensity values captured with different photosensitive elements of a photosensor. These photosensitive elements may be sensitive in a specific spectral range (i.e. representing a specific color or pseudo-color (in NIR)). The image frames include at least some image pixels being representative of a skin portion of the subject. Thereby, an image pixel may correspond to one photosensitive element of a photo-detector and its (analog or digital) output or may be determined based on a combination (e.g. through binning) of a plurality of the photosensitive elements.

In another embodiment the detector comprises one or more optical photoplethysmography sensor(s) 114 (also referred to as contact PPG sensor(s)) configured for being mounted to a skin portion of the subject 120 for acquiring photoplethysmography signals. The PPG sensor(s) 114 may e.g. be designed in the form of a finger-clip or as a wrist-worn wearable device for measuring the blood oxygen saturation or a heart rate sensor for measuring the heart rate, just to name a few of all the possible embodiments.

When using a camera 112 the system 100 may further optionally comprise a light source 140 (also called illumination source), such as a lamp, for illuminating a region of interest 142, such as the skin of the patient's face (e.g. part of the cheek or forehead), with light, for instance in a predetermined wavelength range or ranges (e.g. in the red, green and/or infrared wavelength range(s)). The light reflected from said region of interest 142 in response to said illumination is detected by the camera 112. In another embodiment no dedicated light source is provided, but ambient light is used for illumination of the subject 120. From the reflected light only light in a number of desired wavelength ranges (e.g. green and red or infrared light, or light in a sufficiently large wavelength range covering at least two wavelength channels) may be detected and/or evaluated.

The device 130 is further connected to an interface 150 for displaying the determined information and/or for providing medical personnel with an interface to change settings of the device 130, the camera 112, the PPG sensor(s) 114, the light source 140 and/or any other parameter of the system 100. Such an interface 150 may comprise different displays, buttons, touchscreens, keyboards or other human machine interface means.

A system 100 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between the device 130, the camera 112, the PPG sensor(s) 114 and the interface 150 may work via a wireless or wired communication interface. Other embodiments of the present invention may include a device 130, which is not provided stand-alone, but integrated into the camera 112 or the interface 150.

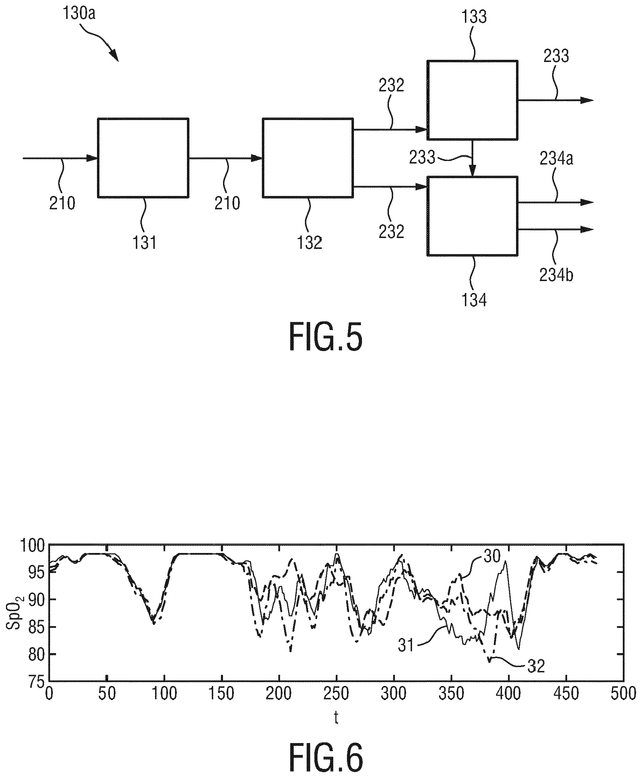

Fig. 5 shows a more detailed schematic illustration of a first embodiment 130a of the device 130 according to the present invention. The device 130a comprises an input interface 131 for obtaining (i.e. retrieving or receiving) at least three detection signals 210 derived from detected electromagnetic radiation transmitted through or reflected from a skin region of the subject 120. The data stream of detection data, i.e. the detection signals 210, is e.g. provided by the camera 112 and/or one or more PPG sensor(s) 114, wherein each detection signal comprises wavelength-dependent reflection or transmission information in a different wavelength channel.

A vital sign extraction unit 132 extracts multiple candidate vital signs 232 of the same type of vital sign from said at least three detection signals 210, wherein each candidate vital sign 232 is extracted from a different detection signal 210 or a different combination of at least two detection signals 210.

A vital sign determination unit 133 determines a final vital sign 233 from said multiple candidate vital signs 232.

A reliability information unit 134 provides reliability information 234a indicating the reliability of said final vital sign 233 and/or one or more of said candidate vital signs 232 and provides unreliability source information 234b indicating the source of unreliability of said final vital sign 233 and/or one or more of said candidate vital signs 232.

The unreliability source information may particularly indicate one or more of subject motion, unexpected blood gasses, blood species and blood composition. All options may change the resulting SpO2 output value. Unexpected blood-composition generally shifts the calibration curve (relation of relative pulsatilities and resulting SpO2 estimate), depending on the unexpected component. Motion also may result in an erroneous SpO2 value, but only if the signal variances due to the motion are relatively large compared to the strength of the pulse-signal. All causes affect the alternative (redundant) measurements in a different way (leading to different errors depending on the wavelength-combination used), while motion specifically can be recognized by the relative large variance in the signal it causes (and the SpO2 values the individual wavelength-combinations trend towards). If the redundant measurements give different SpO2 output, while the signal variance is normal (just pulse), then it can be assumed that there must be unexpected blood components (e.g. CO in the blood of a smoker).

Motion usually is a temporal problem, and waiting for a short while (and withholding alarms) may suffice to get a better measurement a bit later (or a previous valid measurement may temporally be used to bridge the temporally unavailable new reliable value). Unexpected blood gasses may take much longer to fade, and will cause mismeasurements for a long time. In case the subject is near static (low variance signals) it can even be estimated that the concentration of CO, if it is sure that there are no other "pollutants" (e.g. MetHB), or vice-versa if it is known that the subject is a non-smoker and did not suffer CO-poisoning the MetHB level may be measured. Depending on the number of redundant measurements, more blood components may be measured without assuming absence of some.

The various units of the device 13 may be comprised in one or multiple digital or analog processors depending on how and where the invention is applied. The different units may completely or partly be implemented in software and carried out on a personal computer connected to one or more detectors. Some or all of the required functionality may also be implemented in hardware, e.g. in an application specific integrated circuit (ASIC) or in a field programmable gate array (FPGA).

That measurements with two and three wavelengths exhibit differences in behavior during patient movements already becomes evident when analyzing Fig. 1 in more detail. Since the relative pulsatility in the wavelengths shown depends on the oxygenation of the blood, it can be measured using two different wavelength-channels. Different wavelength channels may be sensitive for different wavelength intervals, or for the same wavelength interval, but with a different contribution from individual wavelengths. When using 760nm and 800nm as wavelengths, it can be seen from Fig. 1 that the relative pulsatilities become identical around an arterial oxygenation level of 78%. When using 760nm and 905nm as wavelengths, then the relative pulsatilities become identical around an arterial oxygenation level of about 55% (the point lies outside of Fig. 1). When using three3 wavelengths, e.g. as proposed in the above cited paper of M. van Gastel, S. Stuijk and G. de Haan, "New principle for measuring arterial blood oxygenation, enabling motion-robust remote monitoring", Nature Scientific Reports, Nov. 2016 it can be seen from Fig. 1 that equal pulsatility cannot occur, which makes the system more robust for motion but at the same time leaves the outcome more unpredictable should excessive motion-induced distortions occur.

A system using three wavelengths can achieve four possible outcomes (by three different combinations of two wavelengths and a combination of all three wavelengths) that should be identical in simple cases, while the measurements start deviating if, either motion-distortions are too large, or unexpected blood gasses appear. This is illustrated in Fig. 6 that shows the output from the above mentioned SpO2 measurements during an 8 minutes recording. Signal line 30 shows a measurement using three wavelengths (760nm, 800nm and 842nm). Signal line 31 shows a measurement using the two wavelengths 760nm and 800nm. Signal line 32 shows a measurement using the two wavelengths 760nm and 842nm. In the static part of the protocol (first 2.5 minutes of 8 minutes in total) the measurements agree, thereafter they deviate, indicating a reduced reliability. In the first few minutes the subject is stationary and creates an SpO2-dip by breath-holding for some minute. After about 2.5 minutes the subject starts to move and at about 4 minutes holds his breath again.

From Fig. 6 it can be observed that all three measurements are in agreement for the first 2.5 minutes, both when SpO2 is high and when it dips. In the period thereafter, with significant motion, the three measurements start to deviate indicating a reduced reliability. Since in this experiment the aPBV method was used for all measurements, i.e. the two and three wavelength cases, all methods have some motion robustness. Indeed, the measurement using all three wavelengths most closely approaches the protocol, but motion is too strong to achieve a reliable measurement.

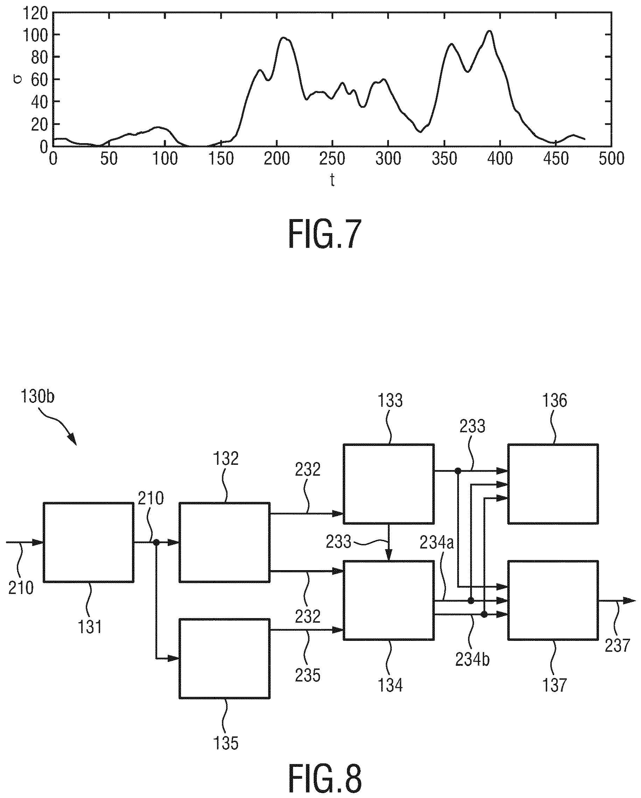

Fig. 7 shows a diagram of the standard deviation $\sigma$ of the three measurements of the SpO2 shown in Fig. 6, clearly indicating a high reliability in the first couple of minutes, and a seriously lower reliability (higher standard deviation) in the second part, while the reliability improves towards the end of the protocol when the subject stops moving and the standard deviation immediately drops.

Briefly summarized, with the present invention, e.g. the device depicted in Fig. 5, a vital sign of a subject is determined. A time signal with at least three channels representing an electromagnetic radiation transmitted through or reflected from a subject's skin is used to extract multiple candidate vital signs from the time signal using combinations of the at least three channels. An indicator of the reliability of at least one of the candidate vital signs and/or a final vital sign and information on the source of any potential unreliability is generated in addition to the final vital sign that is obtained by selecting one of the candidate vital signs or by combining (e.g. averaging, weighted averaging, etc.) two or more candidate vital signs.

Typically, the electromagnetic radiation is in the range of 400nm to 1000nm for pulse, respiration and arterial oxygenation measurement, particularly in the range of 620nm to 920nm. This particular range is most suitable for SpO2 measurement and is attractive for unobtrusive monitoring during sleep (darkness), but if pulse or respiratory signals are required, the visible part of the spectrum may allow a higher quality (i.e. NIR is not necessarily the preferred option in all cases). The detection signals may be acquired by a photo-sensor (array) in direct contact with the skin and/or using a video camera remotely sensing the subject's skin.

An embodiment uses the PBV method to extract a pulse signal (or a respiratory signal) from two or more different combinations of three wavelength channels, e.g. from $[\lambda 1, \lambda 2]$, from $[\lambda 1, \lambda 3]$, and/or from $[\lambda 1, \lambda 2, \lambda 3]$, where $[\lambda 1, \lambda 2, \lambda 3]$ can be the red-green-blue channels of an RGB-video camera, or different wavelength (intervals) selected in the near infrared region, e.g. [760nm, 800nm, 905nm].

In the following some basic considerations with respect to the PBV method shall be briefly explained.

The beating of the heart causes pressure variations in the arteries as the heart pumps blood against the resistance of the vascular bed. Since the arteries are elastic, their diameter changes in sync with the pressure variations. These diameter changes occur even in the smaller vessels of the skin, where the blood volume variations cause a changing absorption of the light.

The unit length normalized blood volume pulse vector (also called signature vector) is defined as PBV, providing the relative PPG-strength in the red, green and blue camera signal, i.e.

$$\vec{P}_{\mathrm{bv}} = \frac{\left[\sigma(\vec{R}_{\mathrm{n}}), \sigma(\vec{G}_{\mathrm{n}}), \sigma(\vec{B}_{\mathrm{n}})\right]}{\sqrt{\sigma^2(\vec{R}_{\mathrm{n}}) + \sigma^2(\vec{G}_{\mathrm{n}}) + \sigma^2(\vec{B}_{\mathrm{n}})}}$$

with $\sigma$ indicating the standard deviation.

To quantify the expectations, the responses $H_{red}(w)$, $H_{green}(w)$ and $H_{blue}(w)$ of the red, green and blue channel, respectively, were measured as a function of the wavelength w, of a global-shutter color CCD camera1, the skin reflectance of a subject, $\rho_s(w)$, and used an absolute PPG-amplitude curve PPG(w). From these curves, shown e.g. in Fig. 2 of the above cited paper of de Haan and van Leest, the blood volume pulse vector PBV is computed as:

$$\vec{P}_{\mathrm{bv}}^T = \begin{bmatrix} \dfrac{\int\limits_{w=400}^{700} H_{\mathrm{red}}(w)I(w)PPG(w)\,dw}{\int\limits_{w=400}^{700} H_{\mathrm{red}}(w)I(w)\rho_s(w)\,dw} \\[20pt] \dfrac{\int\limits_{w=400}^{700} H_{\mathrm{green}}(w)I(w)PPG(w)\,dw}{\int\limits_{w=400}^{700} H_{\mathrm{green}}(w)I(w)\rho_s(w)\,dw} \\[20pt] \dfrac{\int\limits_{w=400}^{700} H_{\mathrm{blue}}(w)I(w)PPG(w)\,dw}{\int\limits_{w=400}^{700} H_{\mathrm{blue}}(w)I(w)\rho_s(w)\,dw} \end{bmatrix}$$

which, using a white, halogen illumination spectrum I(w), leads to a normalized PBV = [0.27, 0.80, 0.54]. When using a more noisy curve the result may be PBV = [0.29, 0.81, 0.50].

The blood volume pulse predicted by the used model corresponds reasonably well to an experimentally measured normalized blood volume pulse vector, PBV = [0.33, 0.78, 0.53] found after averaging measurements on a number of subjects under white illumination conditions. Given this result, it was concluded that the observed PPG-amplitude, particularly in the red, and to a smaller extent in the blue camera channel, can be largely explained by the crosstalk from wavelengths in the interval between 500 and 600 nm. The precise blood volume pulse vector depends on the color filters of the camera, the spectrum of the light and the skin-reflectance, as the model shows. In practice the vector turns out to be remarkably stable though given a set of wavelength channels (the vector will be different in the infrared compared to RGB-based vector).

It has further been found that the relative reflectance of the skin, in the red, green and blue channel under white illumination does not depend much on the skin-type. This is likely because the absorption spectra of the blood-free skin is dominated by the melanin absorption. Although a higher melanin concentration can increase the absolute absorption considerably, the relative absorption in the different wavelengths remains the same. This implies an increase of melanin darkens the skin, but hardly changes the normalized color of the skin. Consequently, also the normalized blood volume pulse PBV is quite stable under white illumination. In the infrared wavelengths the influence of melanin is further reduced as its maximum absorption occurs for short wavelengths (UV-light) and decreases for longer wavelengths.

The stable character of PBV can be used to distinguish color variations caused by blood volume change from variations due to alternative causes, i.e. the stable PBV can be used as a "signature" of blood volume change to distinguish their color variations. The known relative pulsatilities of the color channels PBV can thus be used to discriminate between the pulse-signal and distortions. The resulting pulse signal S using known methods can be written as a linear combination (representing one of several possible ways of "mixing") of the individual DC-free normalized color channels:

$$S = W\,C_n$$

with $WW^T = 1$ and where each of the three rows of the 3 x N matrix $C_n$ contains N samples of the DC-free normalized red, green and blue channel signals $R_n$, $G_n$ and $B_n$, respectively, i.e.:

$$\vec{R}_n = \frac{1}{\mu(\vec{R})}\vec{R} - 1, \quad \vec{G}_n = \frac{1}{\mu(\vec{G})}\vec{G} - 1, \quad \vec{B}_n = \frac{1}{\mu(\vec{B})}\vec{B} - 1.$$

Here the operator $\mu$ corresponds to the mean. Key difference between the different methods is in the calculation of the weighting vector W. In one method, the noise and the PPG signal may be separated into two independent signals built as a linear combination of two color channels. One combination approximated a clean PPG signal, the other contained noise due to motion. As an optimization criterion the energy in the pulse signal may be minimized. In another method a linear combination of the three color channels may be used to obtain the pulse signal.

The PBV method generally obtains the mixing coefficients using the blood volume pulse vector as basically described in US 2013/271591 A1 and the above cited paper of de Haan and van Leest. The best results are obtained if the band-passed filtered versions of $R_n$, $G_n$ and $B_n$ are used. According to this method the known direction of PBV is used to discriminate between the pulse signal and distortions. This not only removes the assumption (of earlier methods) that the pulse is the only periodic component in the video, but also eliminates assumptions on the orientation of the distortion signals. To this end, it is assumed as before that the pulse signal is built as a linear combination of normalized color signals. Since it is known that the relative amplitude of the pulse signal in the red, green and blue channel is given by PBV, the weights, $W_{PBV}$, are searched that give a pulse signal S, for which the correlation with the color channels $R_n$, $G_n$, and $B_n$ equals $P_{bv}$

$$\vec{S}\mathrm{C}_n^T = k\vec{P}_{bv} \Leftrightarrow \vec{W}_{PBV}\mathrm{C}_n\mathrm{C}_n^T = k\vec{P}_{bv}, \qquad (1)$$

and consequently the weights determining the mixing are determined by

$$\vec{W}_{PBV} = k\vec{P}_{bv}\mathrm{Q}^{-1} \text{ with } \mathrm{Q} = \mathrm{C}_n\mathrm{C}_n^T, \qquad (2)$$

and the scalar k is determined such that $W_{PBV}$ has unit length. It is concluded that the characteristic wavelength dependency of the PPG signal, as reflected in the normalized blood volume pulse, PBV, can be used to estimate the pulse signal from the time-sequential RGB pixel data averaged over the skin area. This algorithm is referred to as the PBV method.

According to an embodiment the reliability indicator may output a reliability value (regarding the quality of a pulse, or respiratory signal) depending on the similarity of the candidate pulses, according to a metric (e.g. same peak (pulse-rate) in frequency domain, or similar SNR, or low mean squared difference between pairs of pulse-signals).

In another embodiment the aPBV method is used to extract an SpO2 value from two or more different combinations of three wavelength channels, e.g. from [$\lambda$1, $\lambda$2], from [$\lambda$1, $\lambda$3], and/or from [$\lambda$1, $\lambda$2, $\lambda$3]. In this case, the reliability indicator may output a reliability value depending on the similarity of the SpO2 values, according to a metric (e.g. standard deviation of candidate values (represented in Fig. 7), possibly filtered over a time-interval, and/or the SNR-values of the pulse-signals extracted by the different version of the aPBV method, and/or (in case of a contact sensor) the similarity of the candidate SpO2 values in combination with the strength of a motion signal, e.g. an accelerometer signal, representative for patient motion, and/or (in case of a camera-based sensor) the strength of subject motion as represented by motion-vectors computed from the subject-video).

In the following some basic considerations with respect to the aPBV method shall be briefly explained.

Instead of extracting features from the PPG waveforms, aPBV determines SpO2 indirectly based on the signal quality of the pulse signals extracted with SpO2 'signatures'. This procedure can mathematically be described as:

$$SpO_2 = \underset{SpO_2 \in \mathbf{SpO_2}}{\mathrm{argmax}} \; SNR\left( \overbrace{k\vec{P}_{bv}(SpO_2)[\mathbf{C_n C_n^T}]^{-1} \mathbf{C_n}}^{\vec{W}_{PBV}} \right),$$

$$(3)$$

where $\mathbf{C_n}$ contains the DC-normalized color variations and scalar k is chosen such that $\vec{W}_{PBV}$ has unit length. The SpO2 signatures compiled in $\vec{P}_{bv}$ can be derived from physiology and optics. Assuming identical cameras the PPG amplitudes of N cameras can be determined by:

$$\vec{P}_{bv} = \left\| \begin{bmatrix} \left(\frac{AC}{DC}\right)^1 \\ \left(\frac{AC}{DC}\right)^2 \\ \vdots \\ \left(\frac{AC}{DC}\right)^N \end{bmatrix} \right\| = \left\| \begin{bmatrix} \dfrac{\int_\lambda I(\lambda)F^1(\lambda)C(\lambda)PPG(\lambda)d\lambda}{\int_\lambda I(\lambda)F^1(\lambda)C(\lambda)\rho_s(\lambda)d\lambda} \\ \dfrac{\int_\lambda I(\lambda)F^2(\lambda)C(\lambda)PPG(\lambda)d\lambda}{\int_\lambda I(\lambda)F^2(\lambda)C(\lambda)\rho_s(\lambda)d\lambda} \\ \vdots \\ \dfrac{\int_\lambda I(\lambda)F^N(\lambda)C(\lambda)PPG(\lambda)d\lambda}{\int_\lambda I(\lambda)F^N(\lambda)C(\lambda)\rho_s(\lambda)d\lambda} \end{bmatrix} \right\|.$$

$$(4)$$

Here the PPG amplitude spectrum, PPG($\lambda$), can be approximated by a linear mixture of the light absorption spectra from the two most common variants of the main chromophore in arterial blood, hemoglobin; oxygenated (HbO2) and reduced (Hb):

$$PPG(\lambda) \approx \epsilon_{Hb}(\lambda)c_{Hb} + \epsilon_{HbO_2}(\lambda)c_{HbO_2} = (1 - SaO_2)\epsilon_{Hb}(\lambda) + SaO_2\epsilon_{HbO_2}(\lambda)$$
$$= \epsilon_{Hb}(\lambda) + SaO_2[\epsilon_{HbO_2}(\lambda) - \epsilon_{Hb}(\lambda)],$$

$$(5)$$

where it is assumed that the optical path length differences are negligible for $600 < \lambda < 1000$ nm and $SaO_2 \in [0, 1]$. It is recognized that the wavelength-dependent effect of scattering could render this assumption invalid. When using two wavelengths the ratio-of-ratios parameter R and the ratio of aPBV parameter $\vec{P}_{bv}$ coincide. The wavelength selection may be based on three criteria: 1) the desire to measure oxygen saturation in darkness ($\lambda > 700$nm) for clinical applications, 2) have a reasonable SpO2 contrast, and 3) wavelengths within the spectral sensitivity of the camera. The idea to use three instead of the common two wavelengths used in pulse-oximetry was motivated by the improved robustness of the SpO2 measurement by a factor of two. This can be explained by how motion affects the PPG waveforms when measured with a camera. Since motion-induced intensity variations are equal for all wavelengths, suppression of these artifacts is possible for the aPBV method if the pulse signature $\vec{P}_{bv}$ is not equal to this motion signature, which can be described as a vector with equal weights.

It shall be noted that even if the pulse quality is very good, it does not always mean that the estimated SpO2 value is sufficiently reliable and can be trusted. This may particularly happen when unexpected blood-species (e.g. COHb) are available causing the SpO2 calibration curve to shift, i.e. causing a different signature vector to lead to the optimal pulse quality when using the PBV method or aPBV method for pulse extraction.

Fig. 8 shows a schematic diagram of a second embodiment of a device 130b according to the present invention. In this embodiment additional elements may be provided in addition to the elements of the first embodiment of the device 130a.

In particular, a pulse signal computation unit 135 may be provided that computes multiple pulse signals 235

from said at least three detection signals 210 as explained above. Hereby, each pulse signal 235 may be extracted from a different detection signal or a different combination of at least two detection signals, particularly using the PBV or aPBV method.

Further, an output unit 136, e.g. a display, loudspeaker, computer, etc., may be provided that outputs the final vital sign 233 along with the reliability information 234a and/or the unreliability source information 234b.

Still further, a control unit 137 may be provided that generates, based on the final vital sign 233 and based on the reliability information 234a and/or the unreliability source information 234b, an alarm control signal 237 for controlling an (external) alarm unit configured to issue an alarm and to output the generated alarm control signal 237.

In an embodiment, said control unit 137 is configured to generate an alarm control signal 237 that suppresses an alarm (e.g. low SpO2, stop breathing, abnormal pulse, etc.), at least temporally, in case a low reliability of the vital sign is detected. This may prevent annoying false alarms that in current clinical practice sometimes leads to alarm-fatigue. Such suppression may be most practical if caused by (temporal) movements of the patients, but less practical if due to unexpected blood components as they will take much longer to disappear. Clearly if the unreliability lasts too long, the suppression of an alarm may be dangerous, and how long this temporal suppression may last likely depends on the vital sign and the state of the subject (i.e. may be different at the ICU/NICU than at the general ward) and of the diagnosed cause of the unreliability, i.e. motion vs blood composition.

In another embodiment, said control unit 137 is configured to generate an alarm control signal 237 that triggers an alarm e.g. to indicate that likely unexpected blood-substances (CO, MetHB, etc.) and/or subject motion prohibit proper vital sign measurement. It is known that all SpO2 measurement devices exhibit a miscalibration in case of significant unexpected blood-components, like CO or MetHB, and this function can signal such events to prevent misdiagnosis.

In the latter case, said control unit 137 may use a metric based on dissimilarity of the candidate vital signs in combination with low probability of subject motion as established, e.g. with an accelerometer (contact sensor) or with a motion estimator (e.g. optical flow) operating on a video of the subject or established by interpreting the variance in the different wavelength channels (motion likely causes stronger variance in all wavelength channels). This ensures that the differences between the candidates are not caused by excessive motion but by alternative blood components. Moreover, in case of a camera it can be anticipated that the relative strength (AC/DC) is identical in all wavelength channels. This can also be observed from the individual SpO2 measurements trending towards a pre-known value that depends on the combination of wavelength-channels used.

The above described methods can be applied on detection signals that have been acquired using contact sensors and/or contactless sensors. By way of example, the present invention can be applied in the field of healthcare, e.g. unobtrusive remote patient monitoring, general surveillances, security monitoring and so-called lifestyle environments, such as fitness equipment, fitness/health-watches/bands (typically wrist-worn), or the like. Applications may include monitoring of oxygen saturation (pulse oximetry), heart rate, blood pressure, cardiac output, changes of blood perfusion, assessment of autonomic functions, respiration and detection of peripheral vascular diseases. The present invention can e.g. be used for rapid and reliable pulse detection of a critical patient, for instance during automated CPR (cardiopulmonary resuscitation). The system can be used for monitoring of vital signs of neonates with very sensitive skin e.g. in NICUs and for patients with damaged (e.g. burnt) skin, but may also be more convenient than contact sensors as used in the general ward. Prevention of false alarms and indicators of reliability are crucial for the success of such products.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining at least one vital sign of a subject, said device comprising:

    - an input interface (131) configured to obtain at least three detection signals (210) derived from detected electromagnetic radiation transmitted through or reflected from a skin region of a subject, wherein each detection signal comprises wavelength-dependent reflection or transmission information in a different wavelength channel,
- a vital sign extraction unit (132) configured to extract multiple candidate vital signs (232) of the same type of vital sign from said at least three detection signals, wherein each candidate vital sign is extracted from a different detection signal or a different combination of at least two detection signals,
- a vital sign determination unit (133) configured to determine a final vital sign (233) from said multiple candidate vital signs, and
- a reliability information unit (134) configured to provide reliability information (234a) indicating the reliability of said final vital sign and/or one or more of said candidate vital signs and to provide unreliability source information (234b) indicating the source of unreliability of said final vital sign and/or one or more of said candidate vital signs.

2. Device as claimed in claim 1,
wherein said reliability information unit (134) is configured to provide unreliability source information indicating one or more of subject motion, unexpected blood gasses, blood species and blood composition.

3. Device as claimed in claim 1,
further comprising a pulse signal computation unit (135) configured to compute multiple pulse signals (235) from said at least three detection signals, wherein each pulse signal is extracted from a different detection signal or a different combination of at least two detection signals.

4. Device as claimed in claim 3,
wherein said pulse signal computation unit (135) is configured to compute said multiple pulse signals (235) from said at least three detection signals using a blood-volume vector based PBV or adaptive PBV method.

5. Device as claimed in claim 3,
wherein said pulse signal computation unit (135) is configured to compute said multiple pulse signals (235) from said at least three detection signals using different signature vectors for the computation of each pulse signal, said signature vectors providing an expected relative strength of the pulse signal in the at least three detection signals, wherein the computation of a pulse signal involves a weighted combination at least two detection signals using weights selected such that the resulting pulse signal correlates with the original detection signals as indicated by the respective signature vector.

6. Device as claimed in claim 3,
wherein said reliability information unit (134) is configured to determine said reliability information and said unreliability source information by comparing said pulse signals.

7. Device as claimed in claim 6,
wherein said reliability information unit (134) is configured to determine said reliability information and said unreliability source information based on the similarity of said pulse signals according to a pulse signal similarity metric, in particular in the frequency domain, or according to the SNR, or according to the low mean squared difference between pairs of pulse signals.

8. Device as claimed in claim 1,
wherein said vital sign extraction unit (132) is configured to extract multiple candidate SpO2 values as candidate vital signs from different combinations of at least two detection signals, wherein said vital sign determination unit (133) is configured to determine the final SpO2 value from said multiple candidate SpO2 values, and
wherein said reliability information unit (134) is configured to determine said reliability information and said unreliability source information based on the similarity of said candidate SpO2 values according to an SpO2 similarity metric, in particular according to the standard deviation of candidate SpO2 values, optionally filtered over a time interval, or according to SNR values of pulse signals extracted from said at least two detection signals by different versions of the adaptive PBV method, or according to the similarity of the candidate SpO2 values in combination with the strength of a motion signal representative for subject motion.

9. Device as claimed in claim 1,
further comprising an output unit (136) configured to output the final vital sign along with the reliability information and/or the unreliability source information.

10. Device as claimed in claim 1,

further comprising a control unit (137) configured to generate, based on the final vital sign and based on the reliability information and/or the unreliability source information, an alarm control signal (237) for controlling an alarm unit configured to issue an alarm and to output the generated alarm control signal.

11. Device as claimed in claim 10,
wherein said control unit (137) is configured to generate an alarm control signal (237) that suppresses an alarm in case of low reliability of the final vital sign and/or to generate an alarm control signal (237) that triggers an alarm indicating that unexpected blood gasses and/or subject motion caused a low reliability of the final vital sign.

12. Device as claimed in claim 10,
wherein said control unit is configured to use a metric based on dissimilarity of the candidate vital signs in combination with low probability of subject motion.

13. System for determining at least one vital sign of a subject, said system comprising:

- a detector (110; 112, 114) for detecting electromagnetic radiation transmitted through or reflected from a skin region of a subject and for deriving at least three detection signals (210) from the detected electromagnetic, wherein each detection signal comprises wavelength-dependent reflection or transmission information in a different wavelength channel, and
- a device (130) as claimed in claim 1 for determining at least one vital sign of the subject from the at least three detection signals.

14. Method for determining at least one vital sign of a subject, said method comprising:

- obtaining at least three detection signals (210) derived from detected electromagnetic radiation transmitted through or reflected from a skin region of a subject, wherein each detection signal comprises wavelength-dependent reflection or transmission information in a different wavelength channel,
- extracting multiple candidate vital signs (232) of the same type of vital sign from said at least three detection signals, wherein each candidate vital sign is extracted from a different detection signal or a different combination of at least two detection signals,
- determining a final vital sign (233) from said multiple candidate vital signs, and
- providing reliability information (234a) indicating the reliability of said final vital sign and/or one or more of said candidate vital signs and unreliability source information (234b) indicating the source of unreliability of said final vital sign and/or one or more of said candidate vital signs.

15. Computer program comprising program code means for causing a computer to carry out the steps of the method as claimed in claim 14 when said computer program is carried out on the computer.

## Calibration curve Pbv (normalized)

## FIG.1

## Absorption Spectra

## FIG.2

SpO$_2$ compared to fractional saturation for different
COHb levels with $\lambda_1 = 660$ nm and $\lambda_2 = 940$ nm

FIG.3

FIG.4

130a

133

233

232

232

233

233

210

210

234a

210

234b

131

132

134

## FIG.5

## FIG.6

FIG.7

FIG.8

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2016/253820 A1 (JEANNE VINCENT [US] ET AL) 1 September 2016 (2016-09-01)<br>* paragraph [0009] - paragraph [0010] *<br>* paragraph [0040]; figure 1 *<br>* paragraph [0042] *<br>* paragraph [0062] - paragraph [0063] *<br>----- | 1-15 | INV.<br>A61B5/00<br>A61B5/024<br>A61B5/1455 |
| Y | MARK VAN GASTEL ET AL: "New principle for measuring arterial blood oxygenation, enabling motion-robust remote monitoring", SCIENTIFIC REPORTS, vol. 6, no. 1, 7 December 2016 (2016-12-07), pages 1-16, XP055430882, DOI: 10.1038/srep38609<br>* Noise sensitivity analysis; page 4 *<br>* last paragraph; page 2 *<br>* last paragraph; page 6; figure 7 *<br>* Results; page 3 *<br>* Adaptive BPV method; page 12 - page 13 *<br>----- | 1-15 | |
| A | US 2011/237911 A1 (LAMEGO MARCELO M [US] ET AL) 29 September 2011 (2011-09-29)<br>* paragraph [0037] *<br>* paragraph [0038] - paragraph [0039] *<br>----- | 1-3,8 | |
| A | EP 2 767 232 A1 (KONINKL PHILIPS NV [NL]) 20 August 2014 (2014-08-20)<br>* paragraph [0041] *<br>* paragraph [0048] *<br>----- | 10-12 | |

|  |  |
|---|---|
| | TECHNICAL FIELDS SEARCHED (IPC) |
| | A61B |

The present search report has been drawn up for all claims

| Place of search | Date of completion of the search | Examiner |
|---|---|---|
| The Hague | 16 May 2018 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 9495

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2018

| Patent document cited in search report | | Publication date | Patent family member(s) | | Publication date |
|---|---|---|---|---|---|
| US 2016253820 | A1 | 01-09-2016 | CN | 105636505 A | 01-06-2016 |
| | | | EP | 3057487 A1 | 24-08-2016 |
| | | | JP | 2016534771 A | 10-11-2016 |
| | | | US | 2016253820 A1 | 01-09-2016 |
| | | | WO | 2015055709 A1 | 23-04-2015 |
| US 2011237911 | A1 | 29-09-2011 | NONE | | |
| EP 2767232 | A1 | 20-08-2014 | BR | 112015019314 A2 | 18-07-2017 |
| | | | CA | 2901117 A1 | 21-08-2014 |
| | | | CN | 105007816 A | 28-10-2015 |
| | | | EP | 2767232 A1 | 20-08-2014 |
| | | | EP | 2956061 A1 | 23-12-2015 |
| | | | JP | 2016510243 A | 07-04-2016 |
| | | | RU | 2015139152 A | 17-03-2017 |
| | | | US | 2014235976 A1 | 21-08-2014 |
| | | | WO | 2014125402 A1 | 21-08-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013271591 A1

**Non-patent literature cited in the description**

- **G. DE HAAN ; A. VAN LEEST.** Improved motion robustness of remote-PPG by using the blood volume pulse signature. *Physiol. Meas,* 2014, vol. 35, 1913
- **M. VAN GASTEL ; S. STUIJK ; G. DE HAAN.** New principle for measuring arterial blood oxygenation, enabling motion-robust remote monitoring. *Nature Scientific Reports,* November 2016

- **G. DE HAAN ; A. VAN LEEST.** Improved motion robustness of remote-PPG by using the blood volume pulse signature. *Physiol. Meas.,* 2014, vol. 35, 1913